# EUROPEAN PATENT APPLICATION

(11) **EP 2 127 595 A1**
(43) Date of publication of application: **02.12.2009**
(21) Application number: 09165894.8
(22) Date of filing: 13.09.2000
(51) Int. Cl.: A61B 1/04, A61B 1/313, A61B 1/002

(54) **Miniature endoscope system**

(30) Priority: 13.09.1999 US 153568 P; 28.09.1999 US 156478 P; 06.03.2000 US 187305 P; 06.03.2000 US 518954; 20.06.2000 US 212935 P
(62) Divisional of application: 00961881.0
(71) Applicant: Visionscope Inc., Ashland, MA 01721 (US)
(72) Inventor: Remijan, Paul, Holland, MA 01521 (US); Labombard, Denis, Georgetown, MA 01833 (US)
(74) Representative: Greenwood, John David

(57) **Abstract**

A miniature endoscope (10) for imaging comprising a base unit which includes a handle (16), an imaging device , an imaging channel, an annular illumination channel (34) and in which the imaging device is in the handle (16); the imaging channel (12) is optically coupled to the imaging device and has a first lens and a second lens at the distal end; the annular illumination channel (34) surrounds the imaging channel to provide a probe having a diameter of 2 mm or less.

## Description

### RELATED APPLICATION

This application claims the benefit of U.S. Provisional Application Nos. 60/212,935 filed June 20, 2000, 60/187,305 filed March 6, 2000, 60/156,478 filed September 28, 1999 and 60/153,568 filed September 13, 1999 and is a Continuation-in-Part (CIP) of 09/518,954, filed March 6, 2000, the teachings of which are incorporated herein by reference in their entirety. This application relates to U.S. Application No. 09/520,648 filed March 6, 2000, and U.S. Application No. 09/521,044, filed March 6, 2000, the contents of the above applications are incorporated herein by reference in their entirety.

### BACKGROUND OF THE INVENTION

Endoscopes are devices which allow visual examination inside a hollow cavity. In the field of medicine, the use of endoscopes permits inspection of organs for the purpose of diagnosis, viewing of a surgical site, sampling tissue, or facilitating the safe manipulation of other surgical instruments. Laparoscopes are used particularly for examining organs in the abdominal area. Laparoscopes typically include a light pipe for illuminating the region to be viewed, at least one lens assembly for focusing and relaying the image of the illuminated object, and a housing for the entire assembly which is structured to minimize tissue damage during the surgical procedure. The light pipe can include a fiber optic element for illuminating the site. The laparoscope housing includes a distal section that can be inserted within a body cavity and a proximal section which can include a handle that a user grips to position the distal end near the surgical site.

Existing laparoscopes can include an imaging device such as a charge coupled device (CCD). This device can capture an image of an object being viewed and convey it to a display device, such as monitor. There is a continuing need to improve on the operational features and manufacturability of endoscope systems that improve imaging capability and reduce the risk to the patient.

### SUMMARY OF THE INVENTION

The present invention relates to a small diameter imaging probe or endoscope having improved resolution and field of view. The distal end of the probe, that is inserted into the tissue under examination, is preferably less than 2mm in diameter to reduce trauma at the point of insertion and thereby provide access to sites that are otherwise unavailable for endoscopic examination.

In a preferred embodiment, the endoscope has an optical waveguide or elongated rod, which can be made of a transparent material such as a high refractive index glass, an illumination channel, an optical system and an imaging sensor. The outer diameter of the elongated rod is preferably in the range of 0.6 - 1.6 mm. The imaging device is optically coupled to the rod using one or more lenses.

The waveguide can be used to conduct light from a distal end to a proximal end of the device. The rod can have an outer surface which is coated with an absorbing material or light absorbing layer to inhibit internal reflection and scattering of light. One or more lenses at the distal end of the rod can provide enhanced coupling of light into the distal aperture of the rod.

The illumination channel can surround the rod and transmits light from a light source to an object being examined. The illumination channel is formed with or on the outer surface of the light absorbing layer. A dispersive element can be placed at the distal end of the illumination channel to enhance illumination of the region of interest.

The imaging device can be a charge coupled device (CCD), a CMOS imaging device or other solid state imaging sensor having a two dimensional array of pixel elements. The sensor can capture an image as an object being viewed and transmit it to a computer for storage, processing and/or a display.

In another preferred embodiment, the endoscope has an optical system which includes distal optics and an image relay or tube. The tube can have an inner channel such as a hollow cylinder coated with a light absorbing material to inhibit internal reflection and scattering of light. The endoscope has a duplex configuration which uses a beamsplitter to direct illumination light along the same optical path or air tube used for the transfer of image light from an object being imaged.

The system can use a sheath assembly to provide a sterile barrier over the handle. The barrier can be disposable along with the needle probe.

The light source can be a high power light source. The light can be concentrated by source optics to a polarizer and to a beam splitter before traveling through the tube. The illumination light can be polarized to improve delivery and collection efficiency.

The miniature endoscope system can be used for orthopedic, rheumatologic, general laparoscopic, gynecological or ear, nose and throat procedures, for example. Although many applications require a small diameter to reduce trauma, certain applications can accommodate larger diameters.

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.

### BRIEF DESCRIPTION OF THE DRAWINGS

FIG. 1 illustrates a schematic illustration of a preferred embodiment of the endoscope;
FIG. 2 shows a cross-sectional view of the endoscope optical system;
FIG. 3 illustrates a front view of an embodiment of the endoscope optical system;
FIG. 4 shows a schematic illustration of an alternate embodiment of the endoscope shown in FIG. 1;
FIG. 5 illustrates rectangular optics and a rectangular image transmission rod of an endoscope transmitting light to an imaging device;
FIG. 6 illustrates a super-clad structure integrated over a square or rectangular transmission path of an endoscope;
FIG. 7 illustrates a perspective view of a preferred embodiment of the invention;
FIG. 8 illustrates an endoscope having an air tube and a duplex configuration;
FIGS. 9 and 10 show a side view and a perspective view, respectively, of a miniature endoscope;
FIG. 11 illustrates a rod tip of a miniature endoscope;
FIG. 12 shows a cross-sectional view of a miniature endoscope;
FIG. 13 shows a detailed view of the light transfer and imaging system of the endoscope of FIG. 12;
FIG. 14 illustrates a rod tip of an endoscope mounted within a needle;
FIG. 15 shows a cross-sectional view of an alternate embodiment of an endoscope;
FIG. 16 shows a detailed view of the light transfer and imaging system of the endoscope of FIG. 15;
FIG. 17 shows a micro endoscope with an external light source;
FIG. 18 illustrates an alternate configuration of a lighting system for a miniature endoscope;
FIG. 19 illustrates a cannula for a miniature endoscope; the cannula having an illumination cannula;
FIG. 20 shows a cannula having a stylet;
FIG. 21 is a perspective view of an alternative embodiment of the miniature endoscope;
FIG. 22 is a top sectional view of the miniature endoscope;
FIG. 23 is a side view, a portion shown in hemline of the miniature endoscope;
FIG. 24 is a rear view of the miniature endoscope;
FIG. 25A is a front view of the base of the miniature endoscope with the needle not attached;
FIG. 25B is an enlarged view of a portion of the connection of the endoscope of FIG. 25A;
FIG. 26 is a side sectional view of the miniature endoscope;
FIG. 27A is an enlarged sectional view of a portion of the endoscope of FIG. 26;
FIG. 27B is an enlarged sectional view of the distal end of the endoscope of FIG. 26;
FIG. 28 is a sectional view of the miniature endoscope taken along the line 28-28 of FIG. 26;
FIG. 29A is an enlarged sectional view of a portion of the endoscope of FIG. 28;
FIG. 29B is an enlarged sectional view of a portion of the endoscope of FIG. 28.

### DETAILED DESCRIPTION OF THE INVENTION

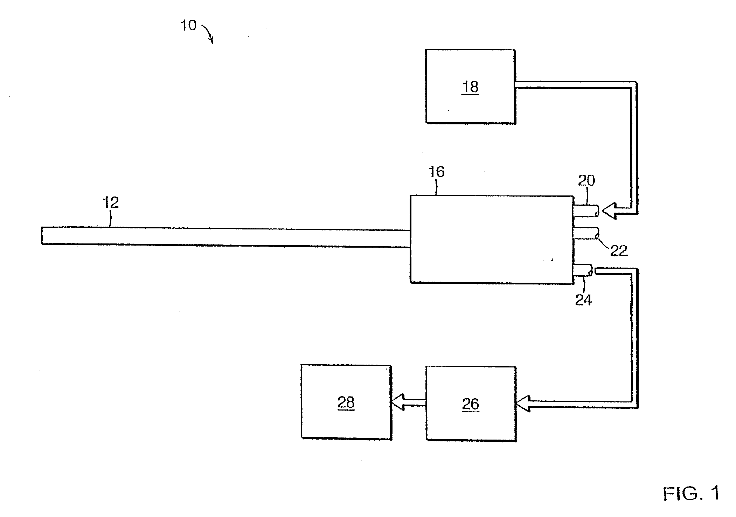

A preferred embodiment of the invention is illustrated in FIG. 1 which shows a miniature endoscope 10. The endoscope 10 has an image transmission path such as an optical waveguide or elongated rod 12 used to view objects to be examined. The elongated rod 12 can be attached to a handle 16. The handle 16 can house a light source input 20 which can connect to a light source 18. In a preferred embodiment, the light source input 20 such as a fiber optic cable optically couples the light source 18 to an illumination channel within the endoscope 10. The handle 16 can also house a power input 22, used to provide power to the endoscope 10. Alternatively, the light source and/or power source can be mounted within the handle.

The handle 16 can also house an image output 24. The image output 24 provides a connection between an imaging device in the endoscope and an electronic storage and/or display device. In one embodiment, the storage device is a computer 26 which is connected to a monitor 28. The imaging device can be a charge coupled device or other pixellated flat panel sensor.

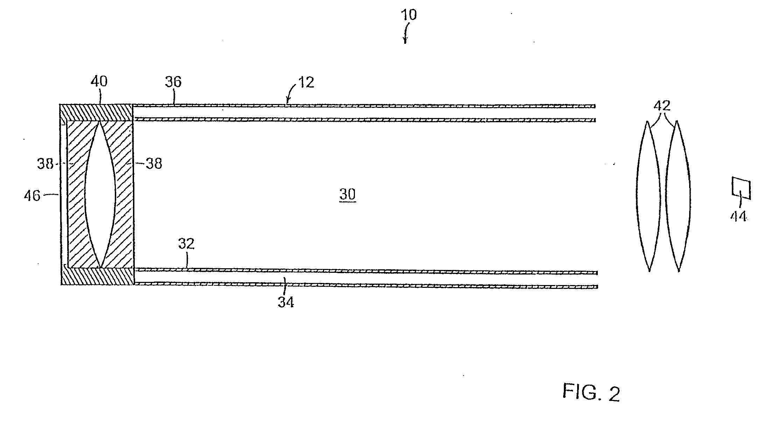

FIG. 2 shows a cross-sectional view of an embodiment of the microendoscope 10. The elongated rod 12 can have a transparent material such as a high index glass rod 30 having a refractive index greater than one, an illumination channel 34, an optical element or distal optics 38 and proximal optics 42.

The distal optics 38 can form a virtual image of an object being examined. In a preferred embodiment, the distal optics 38 can be one or more plastic lenses. The high index glass rod or core 30 links the distal optics 38 to relay optics 42 located in a proximal end of the endoscope 10. In one embodiment, the distal optics comprise two lenses. The high index glass core 30 can have a refractive index of 1.85 and can reduce the optical path between a virtual image created by the distal optics 38 and the relay optics 42. The high index glass rod 30 is preferably free of birefringence to produce an aberration free image at an image sensor 44. Stress within the glass core 30 is necessary for mechanical strength. In a preferred embodiment, the glass core 30 is made of SF57, a pochels glass, which is a glass that can be mechanically stressed without introducing stress birefringence.

The high index glass core 30 can have a tunnel barrier or light absorbing layer or sheath 32. The purpose of the tunnel barrier or sheath 32 is to absorb unwanted light. One option for a tunnel barrier is described in U.S. Patent No. 5,423,312, the entirety of which is incorporated herein by reference. This option employs a glass rod having an outer surface that has been roughened and blackened to provide an absorbing barrier. In contrast, the present invention leaves the glass rod intact and provides an external coating having a lower index of refraction to absorb light crossing the rod's external surface. In a preferred embodiment, the tunnel barrier or absorbing sheath 32 is EMA or extramural absorption glass (available from Shott Fiber Optics, Southbridge, MA). The EMA glass can be extruded during a fiber optics drawing process. The extrusion process leaves the outer surface of the high index glass rod intact. The extruding process instead adds material to the outer surface of the high index glass rod 30 to create a reflective boundary. The extruding process can be performed using a bar in tube drawing process. Similarly, the extruding process can be performed using a differential bar in tube drawing process. In a preferred embodiment, the EMA glass is approximately 5-10 µm thick. The EMA glass can have a refractive index of 1.6, for example.

The illumination channel 34 can be used to provide light from a light source to an object being illuminated. In one embodiment the illumination channel is coupled to glass fiber which is coupled to a light source. In a preferred embodiment, the illumination channel 34 can be extruded during a fiber optics drawing process. In another embodiment, this fiber optic drawing process can be performed in a second drawing process. The illumination channel can have a wall thickness of 0.15mm and can have a refractive index of 1.5 for example.

The image channel or illumination channel 34 can have an outer sheath 36. In a preferred embodiment, the outer sheath 36 is a polyamide coating. The coating can be between 100 and 150 µm thick. The polyamide coating can be applied in a final fiber optics drawing process. Alternatively, one or more of the layers on the rod can be applied by a coating, dipping or deposition process. The polyamide coating can provide strength to the glass core 30. If a glass shatter event were to occur, the polyamide coating can contain the glass from the core 30 to prevent injury to the patient. An outer metal or plastic tube can also be used to enclose the distal end of the device.

The elongated rod 12 can also have a binary phase ring 40 located at its distal end. The ring 40 is positioned on the elongated rod 12 so as to abut the illumination channel 40. The binary phase ring is coupled to the illumination channel in one embodiment. The binary phase ring 40 disperses light traveling through the illumination channel 34 to provide even illumination of the field of view. In a preferred embodiment, the binary phase ring 40 is made from a plastic material. The binary phase ring 40 can also have a distal window 46. The window can be mounted flush against the distal optics 38.

The elongated rod 12 of the endoscope 10 in one embodiment has an outer diameter under 2mm. In another embodiment, the endoscope 10 has an outer diameter of 1.6 mm or less. In a preferred embodiment requiring a small entry site, the endoscope 10 has an outer diameter of 1 to 1.2 mm.

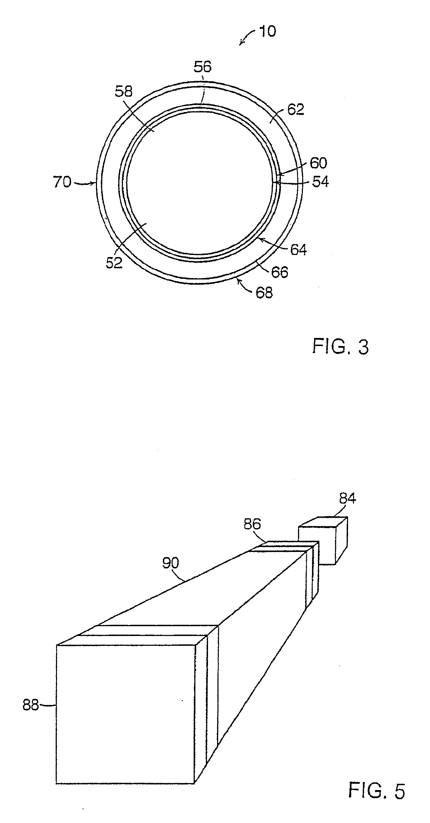

FIG. 3 illustrates a front view of an embodiment of an endoscope 10. The endoscope 10 can have an image light channel 58 and a super-clad structure 68. The image light channel 58 can include and a light absorbing layer 56. The super-clad structure 68 can include a first coating or layer 64, a second coating or layer 66 and an illumination channel 62. The super clad structure 68 directs light through the endoscope 10.

The image light channel 58 can be made from a transparent material or high index glass core 52. In a preferred embodiment, the core 52 is made from a material having a constant refractive index to eliminate deviation of light passing through the material. The constant refractive index may be achieved after the stress of a fiber drawing process by using a pockels glass core, for example. Pockels glasses exhibit zero birefringence when placed in compression or tension. The constant refractive index may also be achieved by annealing the image light channel 58 after the fiber drawing process. The core 52 can also have a first diameter 54. In a preferred embodiment, the first diameter 54 is 1.20 mm.

The light absorbing layer 56 of the image light channel 58, in a preferred embodiment, is a light absorbing glass. The light absorbing layer 56 can have a higher index of refraction than the core 52 and can be made from the same material as the core 52. Light absorbing colorants can be added to the light absorbing glass material to raise its index of refraction and increase its light absorption. In a preferred embodiment, the index of refraction of the light absorbing layer 56 is slightly higher than the index of refraction of the core 52. The light absorbing layer 56 can be applied to the core 52 using a fiber drawing process, for example.

The high index glass core 52 and light absorbing layer 56 can be formed from various types of glass materials. In one embodiment, the image light channel 58 can be formed from an F2 glass core and a BG-4 glass light absorbing layer. The F2 glass core can have a refractive index of 1.620. The BG-4 glass light absorbing layer can have a refractive index of approximately 1.65. In another embodiment, the image light channel 58 can be formed from an F7 glass core and a BG-2 glass light absorbing layer. The F7 glass core can have a refractive index of 1.625. The BG-2 glass light absorbing layer can have a refractive index of approximately 1.66.

The light absorbing layer 56 can have a thickness as low as 5 µm. Preferably, the thickness of the light absorbing layer 56 is no greater than 10 µm. The image light channel 58, formed of the core 52 and the light absorbing layer 56, can have a second diameter 60. In one embodiment, the second diameter 60 is 1.24 mm.

The illumination channel 62 has the first coating 64 and the second coating 66 to form a super-clad structure 68. The first coating 64 is located on an inner surface of the channel 62. The second coating 66 is located on an outer surface of the channel 62. The illumination channel 62 can be made from a high index of refraction material. In one embodiment, the illumination channel 62 can be made from LG1 glass which can have a refractive index of approximately 1.82. Both the first coating 64 and the second coating 66 can be made from a low index of refraction material. In one embodiment, the coatings 64, 66 can be made from EG1 glass which can have a refractive index of approximately 1.50. In another embodiment, the coatings can be made from EG9 glass which can have a refractive index of approximately 1.56. The low index material can provide for illumination containment of the illumination channel 62. The illumination channel 62 can have a thickness of 30 µm. The first 64 and second 66 coating layers can each have a thickness as low as 5 µm, respectively. Preferably, the thickness of each of the first 64 and second 66 coating layers is 10 µm.

The super-clad structure 68 can be made by different processes such as a triple-glass, a tube-extrusion process, a dip coating process or chemical deposition combined with fiber drawing processes, for example.

In one embodiment of a process to fabricate a super-clad structure 68, the image light channel 58 can be exposed to a triple-glass tube-extrusion process, which can form the super-clad structure 68. A bar-in-tube fiber draw can then be used to fuse the super-clad structure 68 around the image light channel 58.

In another embodiment of forming a super-clad structure 68, an image light channel 58 can be dipped in a low index, high temperature polymer to form a first coating 64. A high index plastic can then be extruded over the polymer clad image light channel 58, to form an illumination channel 62. The entire structure can then be dipped in a low index polymer to form the second coating 66.

In another embodiment of a process of fabricating a super-clad structure 68, a metal layer can be chemically deposited onto both sides of an illumination channel 62 to form a super-clad structure 68. In a preferred embodiment, the metal is aluminum. The super-clad structure 68 can then be fused to an image light channel 58 using a bar-in-tube fiber drawing process. The super-clad structure 68 and the image light channel 58, the endoscope 50 can have a third diameter 70. In one embodiment, the third diameter 70 is 1.65mm.

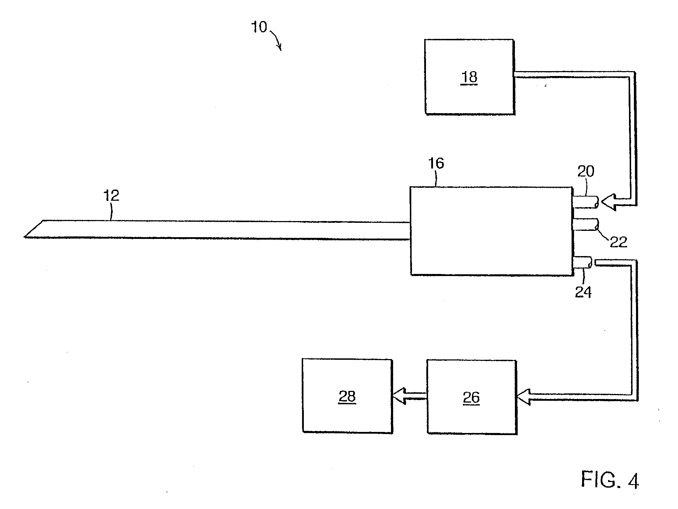

In an alternate embodiment, the endoscope can have an angled distal tip in the shape of a needle shown in FIG. 4. This tip provides for ease of insertion at the site to be examined.

The endoscope can also have square or rectangularly shaped distal optics which can form a virtual image of an object being examined. The endoscope can also have an image transmission path or image channel, such as an elongated rod, which can have a square or rectangularly shaped cross section. Similarly, the endoscope can have square or rectangularly shaped relay optics. By using rectangular optics or a rectangular transmission path, a more efficient transfer of light can be made from an object being viewed to an imaging device, which has a square or rectangular imaging area. All light from an object being imaged can be directly transferred to the imaging area, with little to no light wasted during the transfer.

Generally, endoscopes have circular optics which can transmit light rays to a rectangularly shaped imaging device. For endoscopes having optics with circular cross-sectional areas greater than the cross-sectional area of the imaging device, a portion of the light rays traveling in the arcuate areas of the circular optics will not be transmitted to the imaging device. These light rays can be considered as "wasted" since the light rays fail to intersect the imaging device and are, therefore, unused.

FIG. 5 illustrates rectangular distal optics or optical elements 88 for an endoscope which can transmit light rays to an imaging device 44. In this embodiment, all light rays from the rectangular distal optics 88 can be transferred to the imaging device 84. More light from the object being imaged can therefore be transferred to the imaging device 84 with little waste. A rectangularly shaped transmission path 90 can be used to transfer the light from the distal optics 88 to the imaging device 44. Rectangularly shaped relay optics 86 can also be used to transfer the light from the distal optics 88 to the imaging device 44.

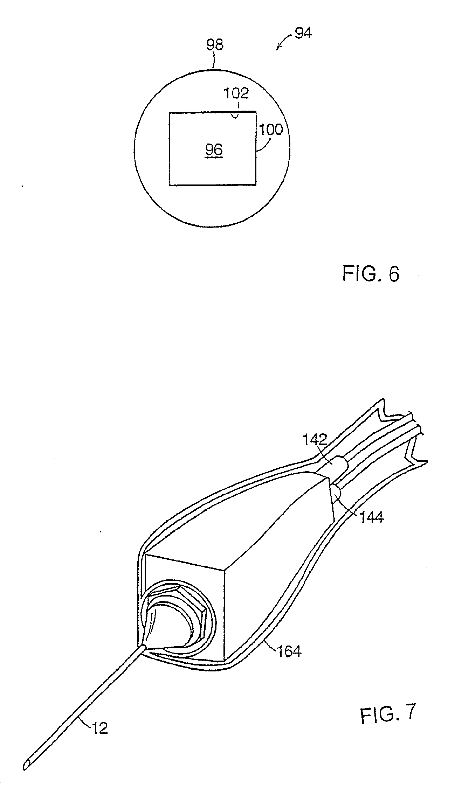

When a square or rectangular transmission path is used in a microendoscope, the inner surface of a super-clad layer of the microendoscope can be shaped to conform to the outer surface of the transmission path. FIG. 6 illustrates a microendoscope 94 having a rectangular light transmission path 96 and a super-clad layer 98. The light transmission path 96 has an outer surface 100 which can be coated with a light absorbing layer which conforms to the geometry of the outer surface 100. When the super-clad layer 98 is to be applied to or extruded over the light transmission path 96, an inner surface 102 of the super-clad layer 98 can conform to the geometry of the light transmission path 96, as illustrated. For a square light transmission path 96, the inner surface 102 of the super-clad layer 98 can be extruded square over the transmission path 96.

FIG. 7 shows a perspective view of a miniature needle endoscope in accordance with the invention. Fiber and electrical cables are connected to the proximal end of handle 16 or needle 12 for insertion into a patient is attached at a distal end of handle 16.

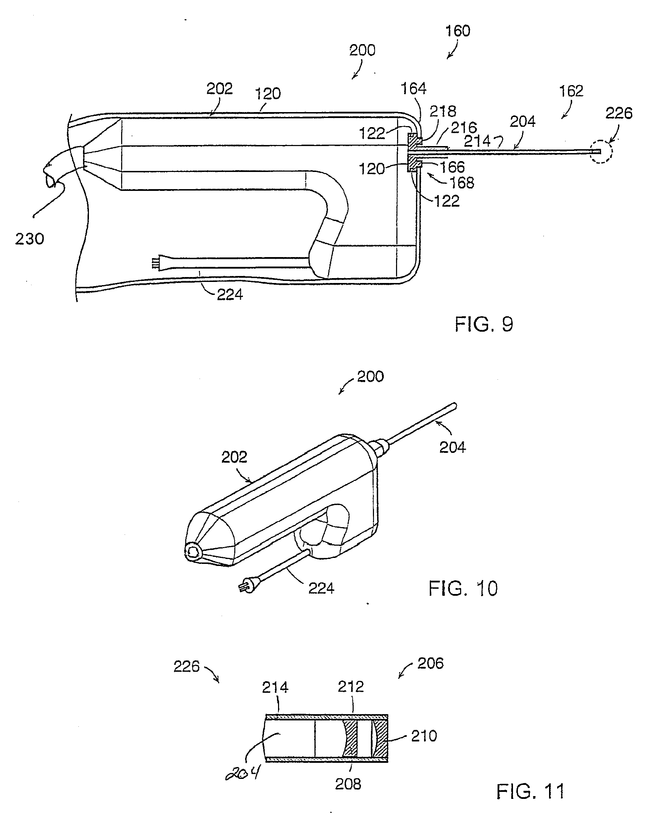

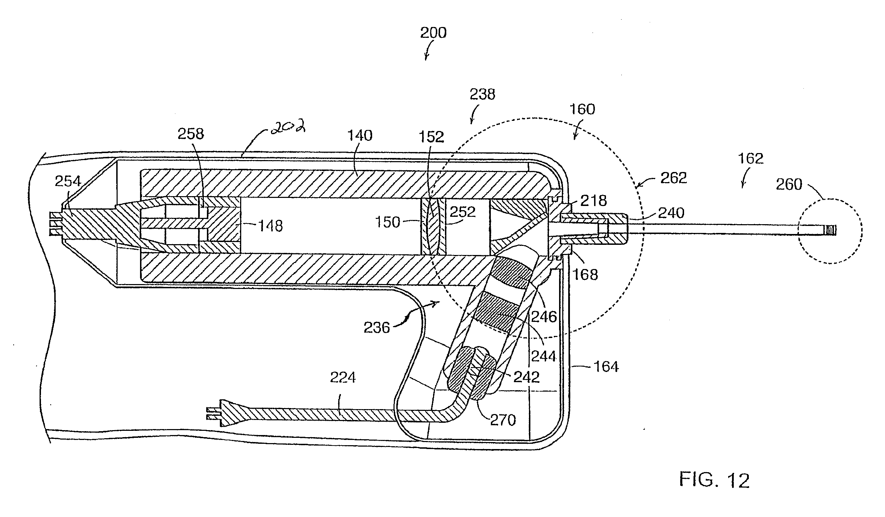

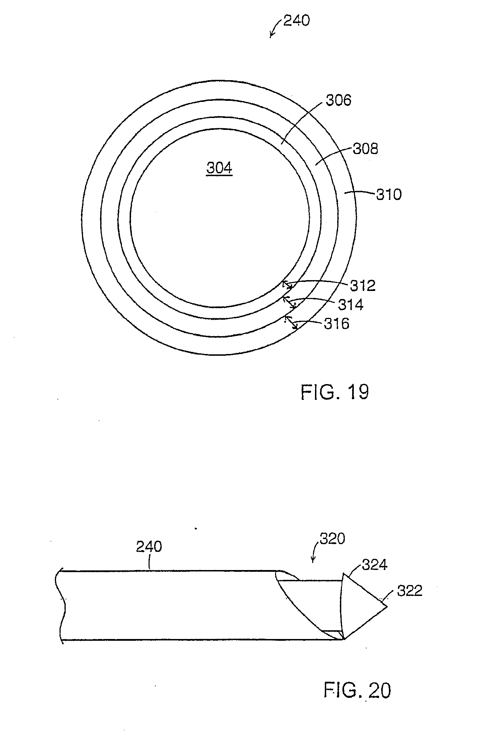

A preferred embodiment of the invention can be considered as three subassemblies. A first subassembly shown in FIG. 9 is the outer handle housing 120 having a distal rod connector 122. A second subassembly is the inner handle 140 shown in FIG. 12. Inner handle 140 includes proximally located fiber and electrical connectors 142 and 144 that are attached to an inner cage assembly 146. The fiber connector 142 connects light from an external source to an illumination annulus 154 which couples light to an illumination channel 308 in needle 240 as shown in FIG. 19. Light collected through needle 240 is coupled to lenses 150 and 152 onto an imaging sensor such as CCD 148.

FIGS. 9 and 12-14 illustrate a disposable third assembly having a rod and needle with a distal lens assembly 162 that is attached to a sterile sleeve assembly 160. The sleeve assembly 160 includes a sleeve 164 that extends over the handle or base unit 202. The distal end of sleeve 164 is secured between plastic frames 166, 170 which can form a mounting hub 218. Frame 166 has a hole 168 that connects to rod and lens assembly 162. Frame 170 connects to rod connector or interface connector 122.

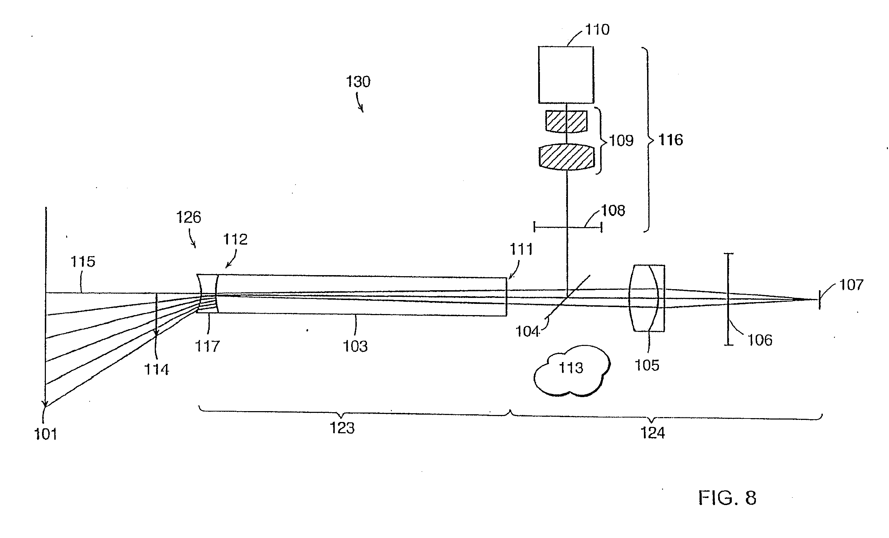

FIG. 8 illustrates an endoscope, identified generally as 130. The endoscope 130 can have an optical system 123 and a handle 124. The optical system 123 can include a tube 103 having a distal end 112, a proximal end 111 and distal optics 117 and can have an outer diameter between 0.6 and 2.0 mm with a preferred outer diameter of about 1.6 mm. The optical system 123 can be disposable. The handle portion 124 can include proximal optics 105, an image polarizer 106, an image sensor 107 and a beam splitter 104. The proximal optics 105 can include an achromatic lens. The beam splitter 104 can be coated with a dielectric coating. The beam splitter coating can be designed to provide maximum reflection of "s polarized" illumination flux and maximum transmission of "p polarized" image light. The curvature of the distal optics 117 can be chosen to minimize retro-reflections of illumination flux appearing in the image.

The endoscope 130 can have a duplex configuration wherein the duplex configuration integrates illumination optics and uses the beam splitter 104 to direct illumination energy along the same optical path used for image light transfer. "Duplex" refers to the optical components and optical path used by illumination flux and image light.

The basic optical components used for both the image light and illumination flux in the endoscope 130 are shown in FIG. 8. As part of the imaging component of the endoscope 130, an object plane 101 can be located from 2 to 20 mm in front of a distal tip 126 of the endoscope 130. The distal optics 117 form a demagnified virtual image 114, located just outside the distal tip 126. A narrow beam of image light from the virtual image 114 can pass through the tube 103, through a dielectric coated beam splitter 104, toward proximal optics 105, and eventually to an image sensor 107, where a real image is formed. Image polarizer 106 can be a linear polarizer that is "crossed" with an illumination polarizer 108 to block retro-reflected illumination flux originating from surfaces of the distal optics.

The tube 103 can be a stainless steel extrusion having a rough inner surface which can be coated with a light absorbing coating, such as spray paint. For example, Krylon #1602, a dull black paint can be used. The tube 103 can have an inner diameter of 1.5 mm with the light absorbing inner wall to reduce or eliminate veiling or scattered light at the image sensor 107. The tube 103 can be filled with air or some other inert gas, or can be evacuated.

The image channel or image relay 103 functions to minimize or absorb unwanted light and hard to image light to prevent veiling glare. The image relay 103 provides high resolution of the optical image, 114 at the plane of the imaging device, removes intermediate image planes and reduces the tolerances needed for optical alignment and optical fabrication. The image relay 103 has an inner tunnel wall that can absorb light diverging from the optics 117. The rough wall surface can disperse up to about 95% or more of unwanted light. The image relay 103 can have a length to diameter (L to D) ratio of between 40:1 and 60:1. The length of the tunnel can be approximately 60 mm. The length of the image relay 103 affects proper illumination of an imaging device, helps control depth of field of view, increases F number for adequate depth of field of view. The image relay 103 can also be disposable.

The optical element or distal optics 117 on the tube 103 can be a polymer lens or an epoxy lens. The distal optics can have a diameter of 1.5 mm. The distal optics 117 can be a single distal lens to reduce retro-reflections. The distal optics 117 can be formed from an epoxy using an injection method. In this method a mandrel can first be placed within the tube 103 from the distal end 112 to the proximal end 111. Epoxy can then be ejected from the needle within 1 mm of the distal end 112 of the tube 103. The epoxy can then be exposed to ultraviolet (UV) light to cure the epoxy. The distal optics 117 can be formed as a concave/negative lens because of the capillary action caused by the air tube 103 after ejection of the epoxy from the needle. The distal 117 and proximal 105 optics can allow control of the size of an image.

The area surrounding the proximal end 111 of the tube can be carefully sculpted and blackened to reduce retro-reflected energy at the image sensor 107 originating from the illumination flux overfill of the air tube 103. The proximal optics 105 are "looking at" this overfill area and the image polarizer 106 can transmit scattered, unpolarized light to the image sensor 107.

The endoscope 130 can be linked via beamsplitter 104 to an illumination system 116. The illumination system 116 can include an illumination source 110 such as a COTS lens end Halogen Lamp having a 0.25 inch diameter from Gilway Technical Lamp. The COTS "Lens End" lamp can have high flux output from a small filament. The illumination source 110 can provide high color temperature visible light for object plane 101 illumination. Source optics 109 can concentrate illumination flux at the proximal end 111 of the tube 103 and provide a low divergence beam to maximize transmission of illumination flux through the tube 103. A beam splitter 104 can redirect illumination flux along an image light axis 115. Illumination polarizer 108 is a linear polarizer oriented to provide "s polarization" at the beam splitter to maximize reflection of illumination flux from dielectric coated beam splitter 104, along axis 115. A light absorbing mechanism or beam dump 113 can remove unused portion of illumination flux from the system to reduce veiling background light that can find its way onto the image sensor.

Illumination optics must be carefully designed to maximize illumination at object plane. The illumination optics create a small spot of light at proximal end of air tube and a collimated beam for maximum transmission through air tube.

Illumination and image polarizers must provide high polarization purity with minimum absorption. For example, dichroic sheet polarizers can be inexpensive, but lossy. Calcite polarizers can be more efficient, but expensive and more difficult to accommodate in a simple optical design.

Unused illumination flux transmitted by the beam splitter must be completely removed from the system because the proximal optics are "looking at" the dump area 113. The image polarizer will transmit scattered, unpolarized light to the image sensor.

All retro-reflections can be minimized using well known "optical isolation" configurations, but not totally eliminated. Therefore, electronic image processing may be required to produce an acceptable image. Since the retro-reflection pattern at the image sensor is unique for each scope, this unwanted light distribution can be recorded for each scope, stored in an image buffer, and subtracted from the video image in real time.

The endoscope 130 can be inserted into a body using a cannula. During an insertion procedure, a cannula can first be inserted into a site within a body. The optical system 123 of the endoscope 130 can then be inserted within the cannula which can have an outer diameter of 1.6 mm. The optical system 123 can pass through the cannula and into the body to provide the user with an image of the site.

The system can be used with a disposable sleeve or sheath to aid in maintaining a sterile environment and reduce the sterilization requirements prior to reuse.

FIGS. 9 and 10 illustrate a miniature endoscope, given generally as 200, in both a side and a perspective view respectively. The endoscope 200 can include a base unit 202 and a sheath assembly 160. The base unit can include a cable 224 which can provide power to an internal light source within the base unit 202. The sheath assembly can include a sterile barrier 164 and a probe or rod and lens assembly 162. The rod and lens assembly 162 can be formed of a rod or waveguide 204 and a object lenses 206. The waveguide can be a hollow channel. The probe can have an annular illumination channel around the waveguide. The probe can have a length between 2 cm and 10 cm. The sterile barrier 164 and the rod and lens assembly 162 can be attached to a mounting hub 218 or second locking element which secures to a first locking element of the base unit 202 of the endoscope 200. The hub 218 can include an interface connection 122 or first locking element that allows the sheath assembly 160 to attach to the base unit 202. The interface connection 122 can be a securing mechanism such as a locking mechanism. The sterile barrier 164 can attach to the mounting hub 218 by bonding. The bonding can include cementing between the sterile barrier 164 and the hub 218, for example. The mounting hub 218 can include a locking mechanism 216 such as a luer lock for example. The locking mechanism 216 can allow connection between the miniature endoscope 200 and a needle such as a 14 gage cannula, for example (manufactured by Popper).

The rod and lens assembly 162 can include a rod tip 226 illustrated in FIG. 11. The rod tip 226 can have object lenses 206. These object lenses can include a first object lens 208 and a second object lens 210. The rod 204 of the rod and lens assembly 162 can be covered by a tube 214 or light absorbing boundary. The tube can be a dark coating in order to reduce or eliminate veiling or scattered light within the rod 204.

The sterile barrier 164 of the sheath assembly 160 can cover the entire base unit 202. This covering provides a sterility of the base unit 202 during a surgical procedure.

The miniature endoscope 200 can be inserted into a cannula or needle 240 as illustrated in FIGS. 12-16. Preferably the needle 240 has a blunt end. The needle can be a 14 gauge needle. To use the miniature endoscope 200 with the needle 26 in a surgical procedure, a sheath assembly 160 can first be placed on a base unit 202. The rod and lens assembly 162 of the sheath assembly 160 can lock into the interface connection 122 of the base unit 202. A needle or cannula 240, having a stylet 320, such as seen in FIG. 20, slidably mounted within the cannula, can be inserted into a surgical site. In the case where a blunt needle or cannula 240 is used, the stylet 320 can cut into the tissue of a surgical site and thereby allow the needle 240 to be inserted into the surgical site. The stylet 320 can then be removed from the cannula 240. The stylet or obturator 320 fills the center portion of the cannula during insertion into a surgical site. The use of the stylet prevents coring of tissue, whereby a cylindrical portion of tissue enters the needle or cannula 240 and can clog the needle cavity. By having a stylet within the needle 240, no such tissue can enter the cannula 240 and can clog the needle cavity.

Once the stylet has been removed from the needle 240, the user can flush the surgical site with saline. Next, the rod and lens assembly 162 of the miniature endoscope 200 can be introduced into the needle 240. The rod portion 204 can be inserted within the needle 240 so that a user can obtain a view of the surgical site. The needle can include a locking mechanism on its proximal end, such as a luer lock for example. The luer lock can attach to the locking mechanism 216 of the mounting hub 218 thereby providing a secure attachment between the endoscope 200 and the needle 240.

FIGS. 12, 13 and 14 illustrate a cross sectional view of the miniature endoscope 200. The endoscope 200 can include a lighting system or light source 236 and an imaging system 238. The lighting system 236 can include a lamp 242, a polarizer 244 and a lens expander 246. The lamp 242 can be mounted within the base unit 202 by a light source housing 270 and can be a high output light source. The polarizer 244 can polarize light from the light source and direct light towards the expander 246. The lens expander 246 can direct light towards a prism 264.

The imaging system 238 of the endoscope 200 can include a first image path lens 150, a second image path lens 152 and a sheet polarizer 252. The imaging system can be mounted within a housing 140. The sheet polarizer 252 can help to eliminate back reflections from the rod and lens assembly 162. The polarizer 252 can have a polarization purity of 10⁻³.

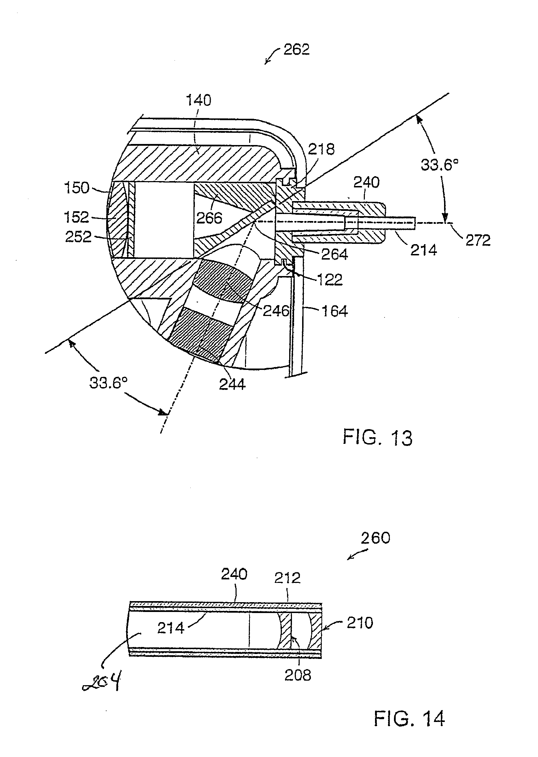

FIG. 13 illustrates a light transfer and imaging system 262 of the endoscope 200 of FIG. 12. The light transfer and imaging system 262 can include a beamsplitter 264 which can be mounted within a housing 266 in the endoscope 200. The beamsplitter 264 can be a prism for example. The beamsplitter 266 can direct light from the lens expander 246 into the rod 204 of the rod and lens assembly 162. This light can be directed at an object to be imaged. The beamsplitter 264 can also receive image light through the rod or channel 204 of an object being imaged and transfer that light to the polarizer 252 of the imaging system 238. The beamsplitter 264 can be mounted within the endoscope 200 at a Brewster's angle with such a mounting. The beamsplitter 264 in this example can form a 33.5° angle with respect to the long axis 272 of the rod. The beamsplitter 264 can also form a 33.5° angle with respect to the central axis of the imaging system 238.

FIG. 12 also illustrates an image sensor 148 mounted within the base unit 202 of the endoscope 200. The image sensor 148 can be mounted within an image sensor housing 258 within the endoscope 200. The image sensor 148 can be attached to an electrical cable connector 254 whereby the cable connector 254 can attach to a cable 230 to provide image signal data from an object being imaged to an external unit. The external unit can be a television screen, for example. The image sensor 148 can be a charge coupled device (CCD). The CCD can be a 1/8 inch CCD. By using a 1/8 inch CCD, the user can quadruple the amount of light he receives from an image. When using a 1/8 inch CCD chip, the focal length of the endoscope 200 can be between 25 and 30 mm. Preferably the focal length is 27 mm.

FIG. 14 illustrates the rod tip 260 of the miniature endoscope 200 whereby the rod tip 260 includes the first object lens 208, the second object lens 210 and a dark coating or tube 214 around a rod 204. As shown, the rod tip 260 is mounted within a needle or cannula 240. Such insertion of the rod tip 260 within the cannula 240 can be done after the cannula 240 is inserted into a surgical site of interest. Once the rod tip 260 is placed in the cannula 240, the cannula 240 can lock on to the base unit 202 by means of a locking mechanism.

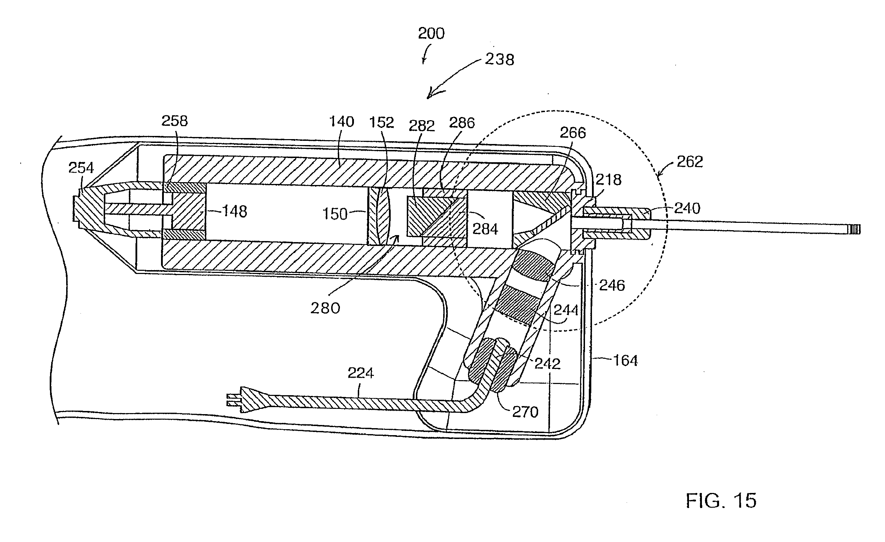

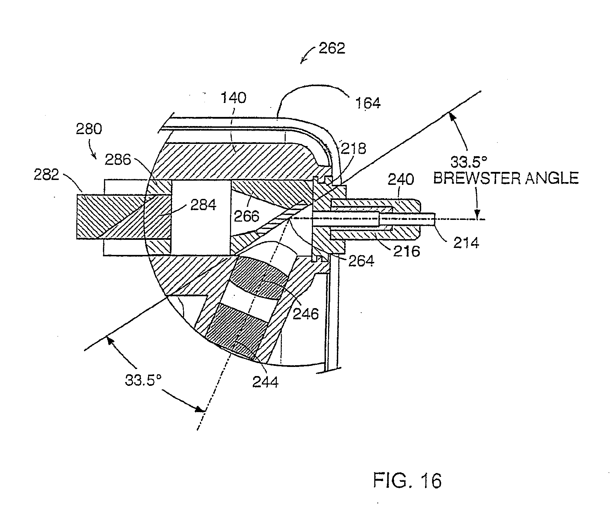

FIGS. 15 and 16 illustrate an alternate to the imaging system 238 illustrated in FIGS. 12, 13 and 14. The imaging system 238 can include a first image path lens 150, a second image path lens 152 and a polarizer 280. The cross polarizer 280 can be made from cal cite and can eliminate back reflections created by the rod and lens assembly 162. The polarization purity of the cross polarizer can be between 10⁻⁵ and 10⁻⁶. The cross polarizer 280 can increase light throughout by 15% to 20%. The polarizer 280 can include a first prism 282 and a second prism 284. The polarizer 280 can be attached to the housing 140 of the endoscope 200 by a polarizer housing 286.

FIG. 16 illustrates the light transfer and imaging system 262 of FIG. 15. Light directed from the lens expander 246 can be sent through the beamsplitter 264 and into the rod 204 to an object being imaged. Light from the object being imaged can be transferred back through the rod 204 and through the prism 264 into the beamsplitter 280. The beamsplitter can transfer the image light to the polarizer 280 which can eliminate back reflections created by the object lenses 206.

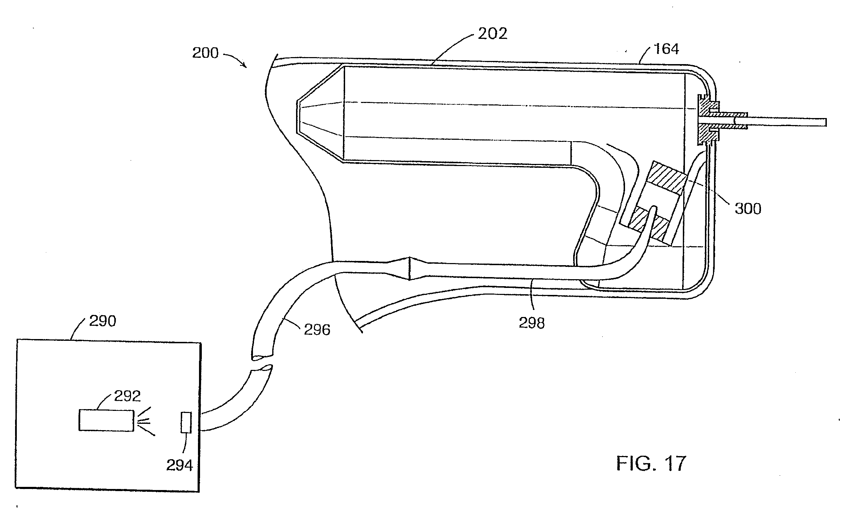

FIG. 17 illustrates a miniature endoscope 200 where the light source of the endoscope 200 is an external light source 290. The external light source can include a lamp 292 and light source optics 294. The lamp 292 can be a xenon lamp which can be 300 watts, for example. The optics 294 and lamp 292 of the external light source 290 can be coupled to the miniature endoscope 200 by a silica cable 296. The endoscope 200 can include a reducer 298 mounted within the base unit 202. The reducer 298 can reduce the cross sectional area of the source by a factor of 2-5 times. Preferably the reducer reduces by a factor of 3.5. When used with a xenon source, the reducer 298 can reduce the aperture size of source for efficient coupling into the probe waveguide. The use of a reducer 298 within the endoscope 200 can simplify the optics within the lighting system 236.

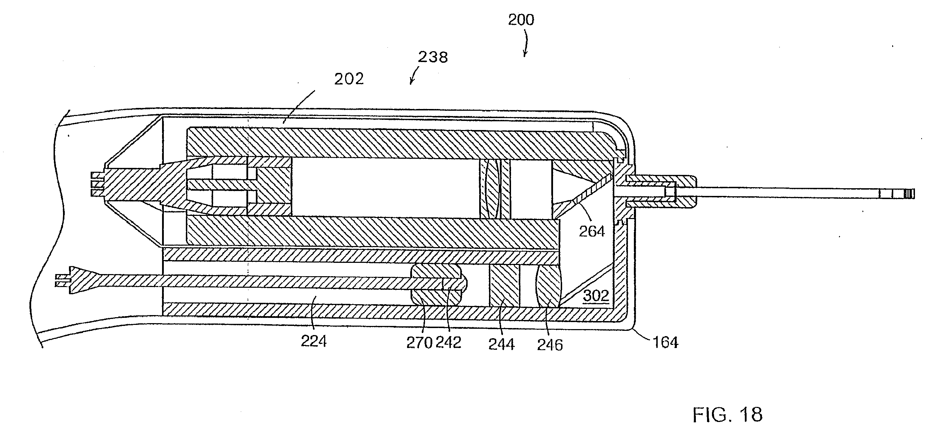

FIG. 18 shows a configuration of the endoscope 200 wherein the lighting system 236 is mounted within the base unit 202 parallel to the imaging system 238. With such a configuration, the lighting system 236 can include a mirror 302. The mirror 302 can be a fold mirror for example. The mirror 302 can be mounted within the endoscope 200 such that light from a light source 242 which travels through a polarizer 244 and an expander 246 can reflect from the mirror to travel to the prism 264.

FIG. 19 illustrates the cross section of a needle 240 wherein the needle acts a reducer to provide light to an object being imaged. The needle 240 can include an aperture 304. The aperture can be surrounded by a first cladding layer 306, an illumination channel 308 and a second cladding layer 310. The first cladding layer can have a first cladding layer thickness 312. The illumination channel 308 can include a channel thickness 314 which can be 10 microns. The second cladding layer 310 can include a second cladding thickness 316 whereby the thickness can be 3 microns.

FIG. 20 illustrates a cannula 240 having a stylet. Prior to inserting a needle 240 into a surgical site, a stylet or obturator can be inserted within the needle 240. The stylet can include a cutting surface 322 and a cleaning edge 324. When the stylet 320 and needle 240 are inserted into a surgical site, tissue can accumulate in an area between the stylet 320 and the needle 240. In order to eliminate this material from the area, the stylet 320 can include a cleaning edge 324 whereby the cleaning edge is formed of a less stiff material than is the cutting edge 322. When the stylet 320 is pulled towards the user after insertion of the needle 240 in the surgical site, the weaker edge or the cleaning edge 324 will bend about the needle thereby cleaning or wiping away any tissue debris from the needle area. Such a cleaning function allows proper insertion of the microendoscope within the cannula and proper viewing of a surgical site.

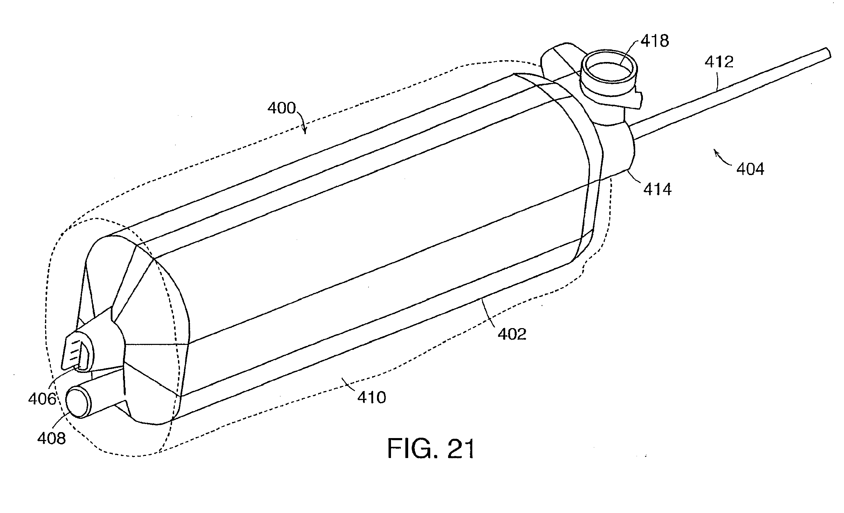

FIG. 21 shows a miniature endoscope 400 in side perspective view. The endoscope 400 includes a base unit 402 and a sheath assembly 404. The base unit 402 includes an electrical connection 406 for the imaging device, such as a CCD and a fiber optic light source connection 408.

The sheath assembly 404 includes a sterile barrier 410 and a rod and lens assembly 412. The sterile barrier 410 and the rod and lens assembly 412 are attached to a mounting hub 414, which is secured to the base unit 402 of the endoscope 400. The mounting hub 414 is a light sheath hub with luer lockside port.

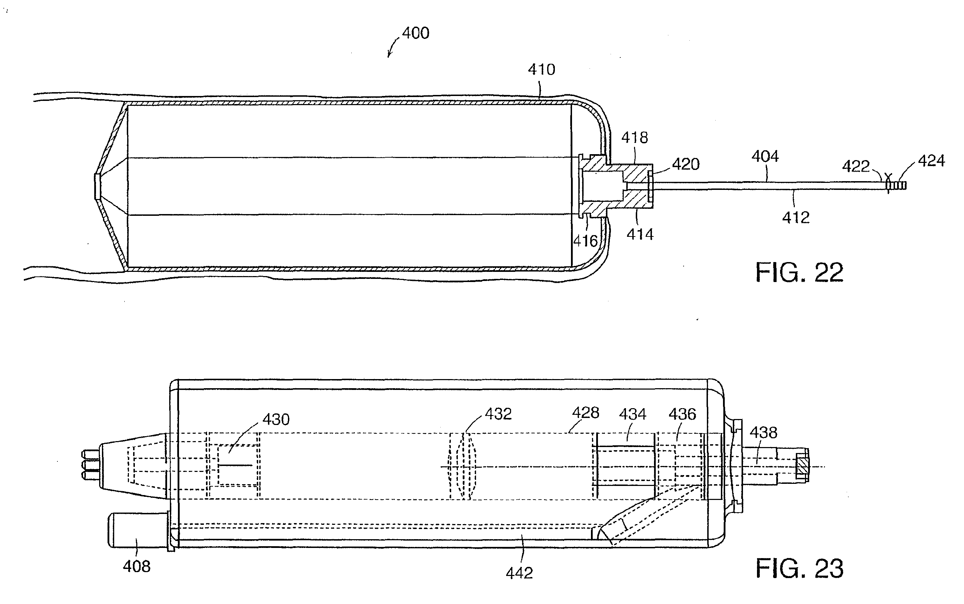

The hub 414 can include an interface connection 416 that allows the sheath assembly 404 to attach to the base unit 402. The interface connection 416 can be a securing mechanism such as a locking mechanism. The sterile barrier 410, as seen in FIG. 22, is attached to the mounting hub 414 by bonding. The bonding can include cementing between the sterile barrier 410 and the hub 414, for example.

The mounting hub 414 can include a locking mechanism 418 such as a luer lock or fitting for example. The locking mechanism 418 can allow connection between the miniature endoscope 400 and a needle such as a 14 gage cannula, for example (manufactured by Popper).

Referring to FIG. 22, a sectional view of the endoscope 400 is shown. The sheath assembly 404 with the rod and lens assembly 412 and sterile barrier 410 is shown. The sterile barrier 410 and the rod and lens assembly 412 are attached to the mounting hub 414. The mounting hub 414 has a fiber optic window 420 which transmits light from a light source to a light sheath in an obturator. The window 420 can be a lens.

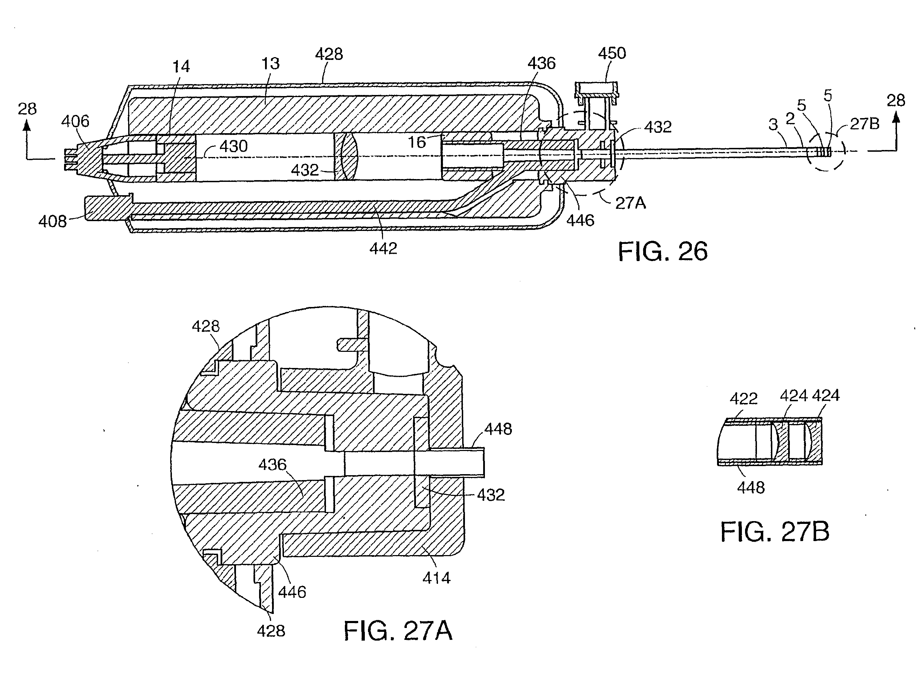

Still referring to FIG. 22, the rod and lens assembly 412 has a darkened outer tube 422 and a pair of object lenses 424. The distal end of the rod and lens assembly 412 will be discussed in further detail with reference to FIG. 27B.

Referring to FIG. 23, the base unit 402 of the endoscope 400 has a main scope body 428 with the CCD camera 430, a set of lenses 432, and a fiber optic tip mount 434 and fiber optic bundle 436 which define an opening 438 through which an optical image passes from the rod and lens assembly 412 towards the CCD camera 430. The opening 438 can be covered by a window or a lens. Still referring to FIG. 23, underlying the main scope by 428 is a fiber optic 442 which extends from the fiber optic light source connection 408 to fiber optic bundle 436.

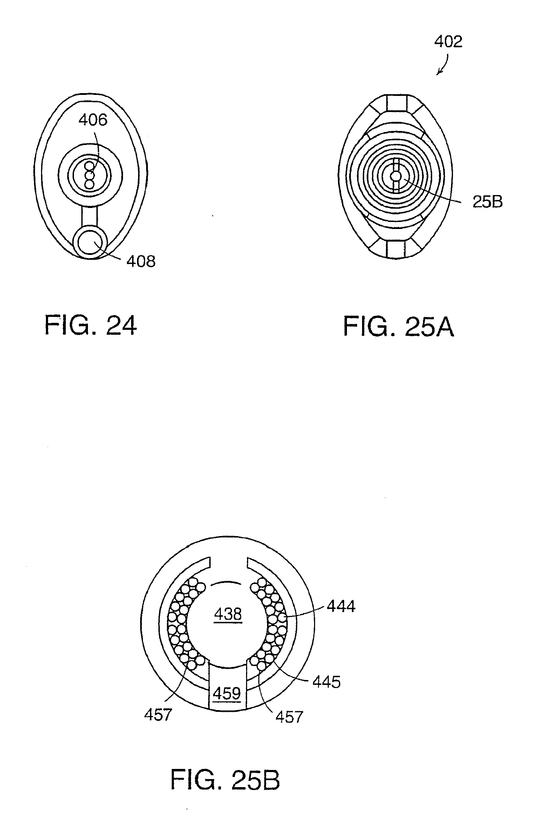

FIG. 24 shows the rear portion of the base unit 402 of the endoscope 400. The electrical connection 406 is seen and in addition the fiber optic light source connection 408 is shown.

Referring to FIG. 25A, a front view of the base unit 402 is shown with the sheath assembly 404 removed. The base unit 402 has a plurality of fiber optic fibers 444 forming an annulus 445 surrounding the opening 438 as seen in FIG. 25B. The fiber optic bundle 436 is formed of these fiber optic fibers 444 in one embodiment. Alternately, the fiber optic bundle 436 has a single fiber optic fiber. The annulus 445 can be a continuous circular pattern. Alternately, the annulus is formed of two semicircular portions 457. A slot 459 can separate the semicircular portions 457. The slot 459 can allow mechanical attachment of the light sheath 422, shown in Figure 27B to the hub 446.

Referring to FIG. 26, a side sectional view of the endoscope 400 is shown. The main scope body 428 as indicated above, has the CCD camera 430 which is connected through the electrical connection 406 to a monitor, such as illustrated in FIG. 1. The CCD camera 430 captures the image projected through the set of lenses 432 that is projected from the high index glass rod of the sheath assembly 404. While the sheath assembly is solid, the image that is projected through the lens 432 in the main scope body is through the opening 438. To light the image, the fiber optics 442 directs the light from the fiber optic light source connection 408 to the fibre optic bundle 436. The fiber optic bundle 436 can be formed of a plurality of fiber optics or from a single fiber optic.

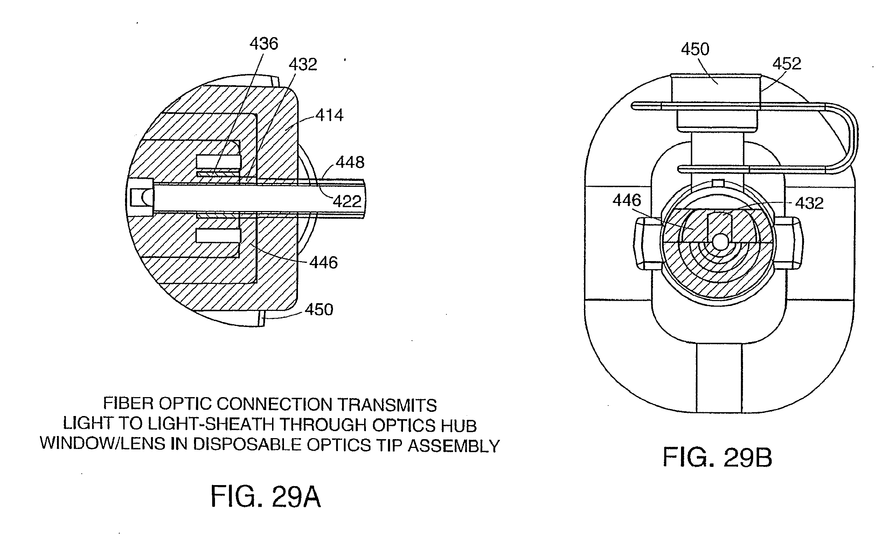

Referring to FIG. 27A, the fiber optic bundle 436 projects its light through the lens 432 into the light sheath 448. The lens 432 can be a window, in an alternate embodiment. The connector between the bundle 436 and the lens 432 is shown in Figure 29A.

The disposable optic tube hub connector 446 with lens 432 can attach to an obturator or needle having a flushing port 450, as shown in Figure 26. The flushing port 450 can include a cap 452. The flushing port 450 allows a user the ability to flush a needle, after insertion into a surgical site, either when the rod and lens assembly 412 is located within the needle or has been removed from the needle. A fluid source, such as a syringe filled with saline, for example, can be attached to the port 450. When a user flushes the needle with saline while the rod and lens assembly 412 is located within the needle, the endoscope can block fluid from flowing from a proximal end of the needle, thereby concentrating flow through a distal end located within a surgical site. Alternately, for a user to flush the needle without the rod assembly 412 within the needle, the cap 452 can be used to cover the proximal end of the needle to direct the flow of the fluid to the distal end of the needle. Such flushing can allow clear viewing of a surgical site.

Referring to FIG. 27B, the distal end of the sheath assembly 404 has the light sheath 448 and encircles the disposable optic dark tube 422 containing the object lenses 424. Light can be transferred from the fiber optic bundle 436 through the light sheath and to an object being imaged.

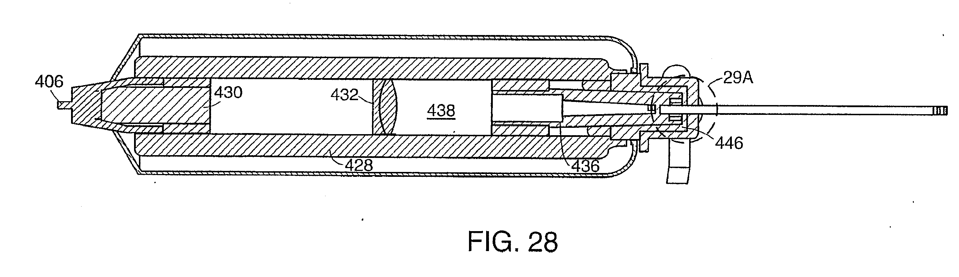

FIG. 28 is a sectional view taken along the line 28-28 of FIG. 26. The figure shows a sectional view of the main scope body 428 cut through and looking up from the optical opening 438. The CCD 430 with connection 406 is shown. Likewise the lens 432 through which the image project are shown.

The fiber optic bundle 436, through which light is passed from the fiber optic 442, as shown in FIG. 26, encircles a portion of the optical opening 438 and directs light through the lens 432 in the disposable optics dark tube hub connector 446 into the light sheath surrounding the rod and lens assembly 412.

FIG. 29A is an enlarged sectional view of the interface of the fiber optic bundle 436, the disposable optics dark tube hub connector 446 and the mounting hub 414. While this invention has been particularly shown and described with references to preferred embodiments thereof, it will be understood by those skilled in the art that various changes in form and details may be made therein without departing from the scope of the invention encompassed by the appended claims.

Embodiments of the present invention may include the features of the following enumerated paragraphs.
1. A miniature endoscope comprising:
   an optical waveguide that transmits an image, the waveguide having:
   a diameter of less than 2 mm, and having a light absorbing layer; an optical element coupled to a distal end of the waveguide;
   an optical relay that is optically coupled to a proximal end of the waveguide; and
   an imaging device mounted at a proximal end of the optical relay that receives an image from the optical waveguide.
2. The miniature endoscope of paragraph 1 wherein the endoscope has an outer diameter of 1.6 mm or less.
3. The miniature endoscope of paragraph 1 wherein the waveguide has an outer diameter between 0.6 and 1.6 mm.
4. The miniature endoscope of paragraph 1 wherein the endoscope further comprises an illumination channel and a binary phase ring which disperses light from the illumination channel.
5. The miniature endoscope of paragraph 1 wherein the waveguide comprises a glass having a refractive index in the range between 1.6 and 1.9.
6. The miniature endoscope of paragraph 1 wherein the waveguide comprises a glass rod.
7. The miniature endoscope of paragraph 1 wherein the light absorbing sheath comprises a thickness between 5 and 10 um.
8. The miniature endoscope of paragraph 1 wherein the light absorbing sheath comprises extramural absorption glass.
9. The miniature endoscope of paragraph 1 wherein the light absorbing sheath comprises a refractive index of 1.6 or less.
10. The miniature endoscope of paragraph 1 wherein the illumination channel has a wall thickness in a range of 0.1 mm and 0.2 mm.
11. The miniature endoscope of paragraph 1 wherein the illumination channel has a refractive index in a range between 1.4 and 1.6.
12. The miniature endoscope of paragraph 1 wherein the illumination channel further comprises an outer sheath.
13. The miniature endoscope of paragraph 12 wherein the outer sheath comprises a polyamide coating.
14. The miniature endoscope of paragraph 13 wherein the polyamide coating has a thickness between 100 and 150 um.
15. The miniature endoscope of paragraph 1 wherein the optical element comprises one or more lenses.
16. The miniature endoscope of paragraph 1 wherein the optical element comprises a plastic lens.
17. The miniature endoscope of paragraph 1 wherein the imaging device comprises a charge coupled device.
18. The miniature endoscope of paragraph 1 wherein the endoscope further comprises a distal needle that penetrates tissue.
19. The miniature endoscope of paragraph 1 wherein the optical element has a rectangular cross-sectional area.
20. The miniature endoscope of paragraph 1 wherein the waveguide has a rectangular cross-sectional area.
21. The miniature endoscope of paragraph 1 wherein the optical relay has a rectangular cross-sectional area.
22. The miniature endoscope of paragraph 1 further comprising a handle in which the optical relay and imaging device are positioned.
23. The miniature endoscope of paragraph 22 further comprising an illumination channel that is optically coupled to a light source in the handle.
24. The miniature endoscope of paragraph 22 further comprising a coupler that connects the handle to the optical waveguide.
25. The miniature endoscope of paragraph 22 further comprising a disposable sheath that extends over the handle.
26. The miniature endoscope of paragraph 25 wherein the sheath is attached to a rigid waveguide housing that is connected to the handle.
27. The miniature endoscope of paragraph 1 further comprising a light source that is optically coupled to the optical waveguide.
28. A miniature endoscope comprising:
   an imaging channel having a diameter less than 2 mm, and a light absorbing layer defining a channel boundary;
   an illumination channel having a first layer on a inner surface of the illumination channel and a second layer on an outer surface of the illumination channel;
   an optical element coupled to a distal end of the imaging channel; an optical relay coupled to a proximal end of the imaging channel;
      and
   an imaging device coupled to a proximal end of the optical relay.
29. The miniature endoscope of paragraph 28 wherein the imaging device comprises a charge coupled device.
30. The miniature endoscope of paragraph 28 wherein the imaging channel comprises a transparent material having a refractive index of at least 1.6.
31. The miniature endoscope of paragraph 30 wherein the imaging light channel comprises a glass rod.
32. The miniature endoscope of paragraph 31 wherein the glass rod comprises an F2 or an F7 glass.
33. The miniature endoscope of paragraph 28 wherein the light absorbing layer comprises a light absorbing glass.
34. The miniature endoscope of paragraph 33 wherein the light absorbing layer comprises a B6-2 glass or a BG-4 glass.
35. The miniature endoscope of paragraph 28 wherein the illumination channel is coupled to a light source.
36. The miniature endoscope of paragraph 28 wherein the illumination channel comprises a material having a refractive index higher than the first layer and the second layer.
37. The miniature endoscope of paragraph 28 wherein the illumination channel comprises a transparent material having a refractive index of at least 1.6.
38. The miniature endoscope of paragraph 28 wherein the first layer and the second layer each have index of refraction of less than 1.6.
39. The miniature endoscope of paragraph 28 wherein the endoscope has a handle and a rigid channel having connected to the handle with a coupler.
40. The miniature endoscope of paragraph 28 wherein the optical element has a rectangular cross-sectional area.
41. The miniature endoscope of paragraph 28 wherein the imaging channel has a rectangular cross-sectional area.
42. The miniature endoscope of paragraph 28 wherein the optical relay has a rectangular cross-sectional area.
43. A method of forming a reflective boundary on a glass channel for a micro endoscope comprising the steps:
   providing a glass channel for a microendoscope; providing a light absorbing material;
   extruding the light absorbing material over the glass channel to form a reflective boundary on the glass channel.
44. The method of paragraph 43 further comprising the step of using a fiber optic drawing process to extrude the light absorbing material over the glass channel.
45. The method of paragraph 43 further comprising the step of using a bar-in-tube drawing process to extrude the light absorbing material over the glass channel.
46. A method of forming an image light channel for a microendoscope comprising the steps:
   providing an illumination channel having a refractive index;
   coating an inner surface and an outer surface of the illumination channel with a material having a refractive index lower than the illumination channel refractive index;
   providing an image light channel for a microendoscope; and attaching the illumination channel to the image light channel.
47. The method of paragraph 46 further comprising the step of using a tube extrusion process for form the coatings on the illumination channel.
48. The method of paragraph 46 further comprising the step of depositing a glass on the outer surface and the inner surface of the illumination channel.
49. The method of paragraph 46 further comprising the step of using a bar-in-tube fiber drawing process to fuse the illumination channel to the image light channel.
50. A method of forming a cladding structure on an image light channel for a microendoscope comprising the steps:
   providing an image light channel;
   forming a material having an index of refraction on the image light channel that is lower than the index of refraction of the image light channel to form a first cladding;
   extruding an illumination channel over the first cladding on the image light channel; and
   forming a second cladding on the illumination channel.
51. A miniature endoscope comprising:
   a handle having an imaging device, a light source and a first coupling element;
   a rigid probe having a waveguide and a second coupling element that connects the probe to the first coupling element, the waveguide having a light absorbing boundary.
52. The endoscope of paragraph 51 further comprising:
   a beamsplitter mounted within the housing wherein the beamsplitter directs light from the lighting source through a rod and lens assembly to an object such that the beamsplitter receives light from an object through the rod and lens assembly and directs the light to the imaging device.
53. The endoscope of paragraph 51 wherein the waveguide comprises a hollow channel.
54. The miniature endoscope of paragraph 51 wherein the imaging device comprises a plurality of lenses and a polarizer.
55. The miniature endoscope of paragraph 54 wherein the polarizer comprises a sheet polarizer.
56. The miniature endoscope of paragraph 54 wherein the polarizer comprises a cross polarizer.
57. The miniature endoscope of paragraph 56 wherein the cross polarizer comprises a first prism and a second prism.
58. The miniature endoscope of paragraph 51 wherein the light source is coupled to a polarizer and a lens.
59. The miniature endoscope of paragraph 51 wherein the light source is coupled to an illumination channel with a fiber optic element.
60. The miniature endoscope of paragraph 51 wherein the probe comprises an annular illumination channel around the waveguide.
61. The miniature endoscope of paragraph 51 wherein the light source comprises an external lamp.
62. The miniature endoscope of paragraph 61 wherein the external lamp comprises a xenon light source.
63. The miniature endoscope of paragraph 51 wherein the endoscope further comprises a sheath attached to the probe and extending over the handle.
64. The miniature endoscope of paragraph 53 wherein the sheath comprises a sterile barrier.
65. The miniature endoscope of paragraph 51 wherein the probe comprises a needle with a distal optical system.
66. The miniature endoscope of paragraph 51 further comprising a cannula wherein the probe fits within the cannula.
67. The miniature endoscope of paragraph 51 wherein waveguide comprises a rod and lens assembly having a locking mechanism wherein the cannula locks onto the rod and lens assembly.
68. The miniature endoscope of paragraph 66 wherein the cannula comprises an illumination channel.
69. The miniature endoscope of paragraph 66 wherein the cannula further comprises a stylet.
70. A method of using a miniature endoscope comprising:
   providing a base unit and a sheath assembly having a probe waveguide and a sterile barrier; and
   attaching the sheath assembly to the base unit such that the sterile barrier extends over the base unit.
71. The method of paragraph 70 further comprising providing a cannula and securing the cannula to the sheath assembly.
72. The method of paragraph 70 further comprising providing a luer fitting on the sheath assembly.
73. The method of paragraph 70 further comprising disposing of the sheath assembly after use and attaching a second sheath assembly to the base unit for further use.
74. The method of paragraph 70 further comprising providing a probe waveguide having a hollow channel and a light absorbing channel wall.
75. The method of paragraph 70 further comprising providing a base unit including a handle, an imaging device within the handle and a relay optical system that couples an image from the waveguide to the imaging device.
76. The method of paragraph 70 further comprising providing a probe waveguide having a diameter of 2 mm or less.
77. The method of paragraph 70 further comprising connecting the base unit to a display.
78. The method of paragraph 70 further comprising providing an annular illumination channel in the probe.
79. The method of paragraph 70 further comprising providing a probe waveguide having a length between 2 cm and 10 cm.
80. The method of paragraph 70 further comprising directing polarized light through the waveguide.

## Claims

1. A disposable sheath for a miniature endoscope comprising:
a sheath assembly having an imaging channel, an annular illumination channel and a connector that can connect a proximal end of the sheath assembly and in which:
the annular illumination channel surrounds the imaging channel to provide a probe having a diameter of 2 mm or less, the illumination channel having an optical coupler.

2. The disposable sheath of claim 1 and any of:
a) further comprising a cannula connectable to the sheath assembly, and, optionally, further comprising a fluid port on the cannula for fluid delivery and/or wherein the cannula comprises a metal tube;
b) wherein the sheath has a length between 2cm and 10cm;
c) wherein the annular illumination channel comprises a fiber optic device;
d) further comprising a first lens and a second lens at a distal end of the imaging channel;
e) further comprising a sterile barrier attached to the sheath, in which case, optionally, wherein the sterile barrier is attached to a proximal end of the disposable sheath and can extend over an endoscope handle attached to the proximal end of the probe;
f) wherein the optical coupler can optically connect to a light source in an endoscope handle;
g) wherein the probe has a length for insertion within an orthopedic region of a patient;
h) wherein the probe has a diameter of 1.6 mm or less;
i) wherein the connector attaches the sheath assembly to an endoscope handle;
j) further comprising a relay lens at a proximal end of the imaging channel that optically couples an image to an imaging device in an endoscope handle.;
k) further comprising a hub assembly with the optical connector that optically couples the illumination channel to a light source located within an endoscope handle.
l) wherein the annular illumination channel is between an inner tube and an outer sheath;
m) wherein the annular illumination channel has an outer plastic sheath; n) wherein the annular illumination channel has an outer metal tube; and 0) wherein the optical coupler comprises a fiber optic cable connector.

3. A miniature endoscope for imaging comprising:
a base unit which includes a handle, an imaging device, an imaging channel, an annular illumination channel and in which:
the imaging device is in the handle;
the imaging channel is optically coupled to the imaging device and has a first lens and a second lens at the distal end;
the annular illumination channel surrounds the imaging channel to provide a probe having a diameter of 2 mm or less.

4. The endoscope of claim 3 and any of:
a) further comprising a cannula secured to a detachable sheath assembly including the imaging channel and the annular illumination channel, and, optionally, further comprising a luer fitted on the cannula;
b) including a relay optical system that couples an image from the waveguide to the imaging device;
c) the probe having a length between 2cm and 10cm;
d) further comprising a light source, such as a lamp, in the handle;
e) further comprising an external light source connected to the handle with a fiber optic cable;
f) wherein the handle comprises a distal end and a proximal end having a cable connector that connects to a cable that transmits image data from the handle to an electronic storage device, such as a computer, and, optionally, further comprising a display device that displays orthopedic images recorded by a flat panel imaging device, such as CCO or CMOS imaging device in the handle, in which case, further optionally, wherein the imaging device has at least 300,000 pixels;
g) wherein the imaging channel and the illumination channel can be detached from the handle and, optionally, wherein the illumination channel comprises a disposable device, in which case, further optionally, further comprising a connector that attaches the illumination channel to the handle;
h) wherein the probe has a size for insertion within an orthopedic region of the body for orthopedic imaging;
i) further comprising a sterile barrier attached to the probe that can extend over the handle;
j) wherein a numerical aperture of illumination light is matched to a numerical aperture of the imaging channel;
k) further comprising a syringe coupled to a fluid port to deliver a fluid into a site within the body or to flush the site; and
l) further comprising an image sensor housing in which the imaging device is positioned, the image sensor housing being attached to an endoscope body having a plurality of relay lens that couple light from the detachable image channel to the imaging device.

5. A method of fabricating a disposable sheath for an endoscope comprising:
fabricating a fiber optic device to form an annular illumination channel; and forming an outer sheath on the fiber optic device having a diameter of 2 mm or less, attaching an inner tube to the annular illumination channel having an inner tube.

6. The method of claim 5 further comprising using a fiber optic drawing process to form the annular illumination channel.

7. The method of claim 5 further comprising forming a plastic outer sheath.

8. The method of claim 5 further comprising a channel within the inner tube for an imaging channel.

9. The method of claim 5 further comprising forming an outer sheath having a size for insertion within a cannula.

10. The method of claim 9 further comprising a cavity between the outer sheath and the cannula tube for passage of a fluid through a fluid port.

11. The method of claim 5 further comprising attaching an optical coupler to the proximal end of the illumination channel for coupling to a light source.

12. The method of claim 5 further comprising applying a coating to the fiber optic device to form the outer sheath.

13. The method of claim 5 further comprising attaching a mechanism to a proximal end of the sheath to mount to an endoscope handle.

14. The method of claim 5 further comprising attaching a window to a distal end of the sheath to enclose an imaging tube, the window positioned within the annular illumination at the distal end of the sheath.

15. The method of claim 5 and any of:
a) wherein the inner tube comprises a metal;
b) wherein the outer sheath comprises a metal; and
c) further comprising forming the imaging channel with at least one lens at a distal end.
